# FASCICULE DE BREVET EUROPEEN

(11) **EP 3 063 299 B1**
(45) Date de publication et mention de la délivrance du brevet: **27.02.2019**
(21) Numéro de dépôt: 14809446.9
(22) Date de dépôt: 31.10.2014
(51) Int. Cl.: C12Q 1/6888, C12Q 1/6879

(54) **PROCÉDÉ ET KIT D'IDENTIFICATION D'UN CHIEN PAR L'ANALYSE D'UN ÉCHANTILLON BIOLOGIQUE**
VERFAHREN UND KIT ZUR IDENTIFIZIERUNG EINES HUNDES BEI ANALYSE EINER BIOLOGISCHEN PROBE
PROCESS AND KIT FOR THE IDENTIFICATION OF A DOG BY ANALYSIS OF A BIOLOGICAL SAMPLE

(30) Priorité: 31.10.2013 FR 1360713
(43) Date de publication de la demande: 07.09.2016
(73) Titulaire: Doutremepuich, Christian, 33200 Bordeaux (FR)
(72) Inventeur: BEAUFILS, Martine, F-33230 Les Eglisottes (FR); DOUTREMEPUICH, Christian, F-33700 Mérignac (FR)
(74) Mandataire: IP Trust
(86) Numéro de dépôt international: PCT/FR2014/052776
(87) Numéro de publication internationale: WO 2015/063429

(56) Documents cités:
- WO-A2-00/29615
- US-A1- 2004 146 915
- US-A1- 2008 295 191
- US-A1- 2013 071 847
- B. BERGER ET AL: "Validation of two canine STR multiplex-assays following the ISFG recommendations for non-human DNA analysis", FORENSIC SCIENCE INTERNATIONAL: GENETICS, vol. 8, no. 1, 7 septembre 2013 (2013-09-07), pages 90-100, XP055125235, ISSN: 1872-4973, DOI: 10.1016/j.fsigen.2013.07.002
- BRADLEY K. TOM ET AL: "Development of a Nomenclature System for a Canine STR Multiplex Reagent Kit", JOURNAL OF FORENSIC SCIENCES, vol. 55, no. 3, 1 mai 2010 (2010-05-01), pages 597-604, XP055125237, ISSN: 0022-1198, DOI: 10.1111/j.1556-4029.2010.01361.x
- GÜNTHER SCHARNHORST ET AL: "An assessment of scientific and technical aspects of closed investigations of canine forensics DNA - case series from the University of California, Davis, USA", CROATIAN MEDICAL JOURNAL, vol. 52, no. 3, 1 juin 2011 (2011-06-01), pages 280-292, XP055125258, ISSN: 0353-9504, DOI: 10.3325/cmj.2011.52.280
- "Reportage TV7 mallette Hygène", , 6 octobre 2014 (2014-10-06), XP054975792, Extrait de l'Internet: URL:http://www.animagene.fr/actualites-ana lyse-genetique-adn-animale/actualites-hyge ne/reportage-tv7/ [extrait le 2015-03-18]
- A. AASPÕLLU ET AL: "The first criminal case in Estonia with dog's DNA data admitted as evidence", INTERNATIONAL CONGRESS SERIES, vol. 1239, 1 janvier 2003 (2003-01-01), pages 847-851, XP055125230, ISSN: 0531-5131, DOI: 10.1016/S0531-5131(02)00224-8
- BERGER C ET AL: "Sequence analysis of the canine mitochondrial DNA control region from shed hair samples in criminal investigations", METHODS IN MOLECULAR BIOLOGY, HUMANA PRESS, INC, US, vol. 830, 1 janvier 2012 (2012-01-01), pages 331-348, XP009178683, ISSN: 1940-6029, DOI: 10.1007/978-1-61779-461-2_23
- Anonymous: "AmpFlSTR COfiler PCR Amplification Kit", Applied Biosystems , 1 août 2012 (2012-08-01), XP055125232, Extrait de l'Internet: URL:https://www3.appliedbiosystems.com/cms /groups/applied_markets_support/documents/ generaldocuments/cms_041009.pdf [extrait le 2014-06-25]
- TERI KUN ET AL: "Developmental validation of Mini-DogFiler for degraded canine DNA", FORENSIC SCIENCE INTERNATIONAL: GENETICS, vol. 7, no. 1, 4 octobre 2012 (2012-10-04) , pages 151-158, XP055125256, ISSN: 1872-4973, DOI: 10.1016/j.fsigen.2012.09.002
- YVONNE MARSCHALL ET AL: "Mapping quantitative trait loci for canine hip dysplasia in German Shepherd dogs", MAMMALIAN GENOME, SPRINGER-VERLAG, NE, vol. 18, no. 12, 20 novembre 2007 (2007-11-20), pages 861-870, XP019561881, ISSN: 1432-1777, DOI: 10.1007/S00335-007-9071-Z -& Yvonne Marschall ET AL: "Mapping quantitative trait loci for canine hip dysplasia in German Shepherd dogs: online supplementary material", Mammalian Genome, 1 décembre 2007 (2007-12-01), XP055125267, Extrait de l'Internet: URL:http://rd.springer.com/article/10.1007 %2Fs00335-007-9071-z [extrait le 2014-06-26]
- J. ECHEGARAY ET AL: "Noninvasive monitoring of wolves at the edge of their distribution and the cost of their conservation", ANIMAL CONSERVATION, vol. 13, no. 2, 1 avril 2010 (2010-04-01), pages 157-161, XP055043022, ISSN: 1367-9430, DOI: 10.1111/j.1469-1795.2009.00315.x

### SEQUENCE LISTING

<110> ANIMAGENE
<120> PROCEDE ET KIT D'IDENTIFICATION D'UN CHIEN PAR L'ANALYSE D'UN ECHANTILLON BIOLOGIQUE
<130> ANI1
<160> 24
<170> BiSSAP 1.2
<210> 1
   <211> 23
   <212> DNA
   <213> Canis sp.
<220>
   <221> source
   <222> 1..23
   <223> /organism="Canis sp."
   /mol_type="unassigned DNA"
<400> 1
   gaacgcattc ttggtgtggt ctc 23
<210> 2
   <211> 23
   <212> DNA
   <213> Canis sp.
<220>
   <221> source
   <222> 1..23
   <223> /organism="Canis sp."
   /mol_type="unassigned DNA"
<400> 2
   ggccattttt cggcttctgt aag 23
<210> 3
   <211> 22
   <212> DNA
   <213> Canis sp.
<220>
   <221> source
   <222> 1..22
   <223> /organism="Canis sp."
   /mol_type="unassigned DNA"
<400> 3
   gccttattca ttgcagttag gg 22
<210> 4
   <211> 22
   <212> DNA
   <213> Canis sp.
<220>
   <221> source
   <222> 1..22
   <223> /organism="Canis sp."
   /mol_type="unassigned DNA"
<400> 4
   atgctgagtt ttgaactttc cc 22
<210> 5
   <211> 21
   <212> DNA
   <213> Canis sp.
<220>
   <221> source
   <222> 1..21
   <223> /organism="Canis sp."
   /mol_type="unassigned DNA"
<400> 5
   aaatggaaca gttgagcatg c 21
<210> 6
   <211> 21
   <212> DNA
   <213> Canis sp.
<220>
   <221> source
   <222> 1..21
   <223> /organism="Canis sp."
   /mol_type="unassigned DNA"
<400> 6
   ccccttacag cttcattttc c 21
<210> 7
   <211> 20
   <212> DNA
   <213> Canis sp.
<220>
   <221> source
   <222> 1..20
   <223> /organism="Canis sp."
   /mol_type="unassigned DNA"
<400> 7
   gctctttgta aaatgacctg 20
<210> 8
   <211> 21
   <212> DNA
   <213> Canis sp.
<220>
   <221> source
   <222> 1..21
   <223> /organism="Canis sp."
   /mol_type="unassigned DNA"
<400> 8
   gtgggaatcg tcctaaaacc c 21
<210> 9
   <211> 20
   <212> DNA
   <213> Canis sp.
<220>
   <221> source
   <222> 1..20
   <223> /organism="Canis sp."
   /mol_type="unassigned DNA"
<400> 9
   tgcacaccca aaaagtaagc 20
<210> 10
   <211> 21
   <212> DNA
   <213> Canis sp.
<220>
   <221> source
   <222> 1..21
   <223> /organism="Canis sp."
   /mol_type="unassigned DNA"
<400> 10
   caatctgaag ccaatctcat c 21
<210> 11
   <211> 20
   <212> DNA
   <213> Canis sp.
<220>
   <221> source
   <222> 1..20
   <223> /organism="Canis sp."
   /mol_type="unassigned DNA"
<400> 11
   atgagcactg ggtgttatac 20
<210> 12
   <211> 20
   <212> DNA
   <213> Canis sp.
<220>
   <221> source
   <222> 1..20
   <223> /organism="Canis sp."
   /mol_type="unassigned DNA"
<400> 12
   acacaattgc attgtcaaac 20
<210> 13
   <211> 20
   <212> DNA
   <213> Canis sp.
<220>
   <221> source
   <222> 1..20
   <223> /organism="Canis sp."
   /mol_type="unassigned DNA"
<400> 13
   tatcgacttt atcactgtgg 20
<210> 14
   <211> 20
   <212> DNA
   <213> Canis sp.
<220>
   <221> source
   <222> 1..20
   <223> /organism="Canis sp."
   /mol_type="unassigned DNA"
<400> 14
   atggagcctc atgtctcatc 20
<210> 15
   <211> 20
   <212> DNA
   <213> Canis sp.
<220>
   <221> source
   <222> 1..20
   <223> /organism="Canis sp."
   /mol_type="unassigned DNA"
<400> 15
   gtagattaga tctcaggcag 20
<210> 16
   <211> 20
   <212> DNA
   <213> Canis sp.
<220>
   <221> source
   <222> 1..20
   <223> /organism="Canis sp."
   /mol_type="unassigned DNA"
<400> 16
   taggtcctgg tagggtgtgg 20
<210> 17
   <211> 18
   <212> DNA
   <213> Canis sp.
<220>
   <221> source
   <222> 1..18
   <223> /organism="Canis sp."
   /mol_type="unassigned DNA"
<400> 17
   cagccgagca catggttt 18
<210> 18
   <211> 21
   <212> DNA
   <213> Canis sp.
<220>
   <221> source
   <222> 1..21
   <223> /organism="Canis sp."
   /mol_type="unassigned DNA"
<400> 18
   attgattctg atatgcccag c 21
<210> 19
   <211> 20
   <212> DNA
   <213> Canis sp.
<220>
   <221> source
   <222> 1..20
   <223> /organism="Canis sp."
   /mol_type="unassigned DNA"
<400> 19
   ccctctgcct acatctctgc 20
<210> 20
   <211> 21
   <212> DNA
   <213> Canis sp.
<220>
   <221> source
   <222> 1..21
   <223> /organism="Canis sp."
   /mol_type="unassigned DNA"
<400> 20
   tagggcatgc atataaccag c 21
<210> 21
   <211> 20
   <212> DNA
   <213> Canis sp.
<220>
   <221> source
   <222> 1..20
   <223> /organism="Canis sp."
   /mol_type="unassigned DNA"
<400> 21
   ctaagggact gaacttctcc 20
<210> 22
   <211> 20
   <212> DNA
   <213> Canis sp.
<220>
   <221> source
   <222> 1..20
   <223> /organism="Canis sp."
   /mol_type="unassigned DNA"
<400> 22
   gtggaacctg cttaagattc 20
<210> 23
   <211> 21
   <212> DNA
   <213> Canis sp.
<220>
   <221> source
   <222> 1..21
   <223> /organism="Canis sp."
   /mol_type="unassigned DNA"
<400> 23
   catggtctca ggatcctagg a 21
<210> 24
   <211> 24
   <212> DNA
   <213> Canis sp.
<220>
   <221> source
   <222> 1..24
   <223> /organism="Canis sp."
   /mol_type="unassigned DNA"
<400> 24
   ctaagcattc tctctgatgg tctt 24

## Description

### Domaine de l'invention

Le domaine de l'invention est celui de la lutte contre les déjections canines sur la voie publique et l'incivilité des propriétaires de chiens. Le nettoyage des déjections canines est une source considérable de déchets et de dépenses pour les villes. Une ville de quelques millions d'habitants est confrontée à plusieurs milliers de tonnes d'excréments canins par an qu'il est indispensable de ramasser, nettoyer et traiter pour maintenir l'espace public propre et agréable pour les concitoyens. Les déjections canines sur la voie publique sont également à l'origine d'accidents qui peuvent être parfois graves et nécessiter l'hospitalisation de la victime.

Plus spécifiquement, l'invention s'inscrit dans le périmètre de la responsabilisation des propriétaires de chiens par l'identification spécifique de leur chien par analyse génétique d'au moins un échantillon biologique.

### Etat de l'art

Il est connu de l'art antérieur des solutions de ramassage de déjections canines pour les diriger ensuite vers des équipements de traitement des déchets. Il est également connu de l'art antérieur des techniques de filiation de chiens par analyse génétique des liens de parenté entre individus canins.

La demande de brevet US2004/0146915 porte sur des procédés servant à déterminer le sexe d'un sujet canin et consistant à cet effet à mettre en contact un échantillon d'acide nucléique provenant de ce sujet canin avec au moins une sonde ou une amorce spécifique de l'amélogénine canine, et à utiliser la fixation de cette sonde ou de cette amorce pour détecter une différence entre le gène de l'amélogénine canine sur le chromosome Y et le gène de l'amélogénine canine sur le chromosome X, permettant ainsi de déterminer le sexe de dudit sujet canin. Dans certains aspects de cette invention, on détermine le sexe du sujet canin en mettant en contact un échantillon d'acide nucléique avec une paire d'amorces qui génère des produits d'amplification de différentes tailles selon qu'une copie du chromosome X ou du chromosome Y du gène de l'amélogénine canine est amplifiée. Dans certains aspects et modes de réalisation présentés ici, en plus de détecter la fixation d'au moins une sonde ou amorce à un gène d'amélogénine canine, les procédés faisant l'objet de cette invention consistent à détecter la fixation d'au moins une sonde ou amorce sur un locus microsatellitaire canin, fournissant ainsi un test de génotypage et de détermination du sexe.

La demande de brevet internationale WO97/13876 décrit des méthodes de génotypage de chien par analyse des polymorphismes au sein de l'ensemble des séquences de répétition microsatellites d'ADN. On amplifie la séquence de répétition interne au moyen d'amorces spécifiques. Le nombre de répétitions, et par conséquent la distance entre les amorces, varie considérablement au sein d'une population, ce qui permet de disposer d'un marqueur allèle pour un locus donné. Les informations combinées de multiples loci permettent d'effectuer une distinction entre des individus, même au sein de lignées canines consanguines, et elles servent à la vérification de paternité, aux expertises légales et à l'analyse des liens de parenté entre individus.
La demande de brevet US 2013/0071847 A1 porte sur l'identification d'un chien par l'analyse d'un échantillon biologique canin consistant en une déjection canine inconnue abandonnée sur l'espace public. Cette analyse de l'échantillon est comparée avec le profil génétique d'une population connue de chiens enregistrée dans une base de données. La caractérisation des profils génétiques de l'échantillon s'effectuant par des amplifications PCR isolées, mettant chacune en jeu un seul couple d'amorces spécifiques par mix de réaction.

### Inconvénients des solutions de l'art antérieur

Les solutions de l'art antérieur s'attachent à affilier un individu à un rang taxonomique donné (classe, ordre, famille, genre, espèce...) ou à établir un lien de filiation à partir d'une caractéristique génétique précise analysée dans un échantillon biologique prélevé sur l'individu directement ou dans son environnement. Ces solutions utilisent donc des caractéristiques taxonomiques, spécifiques ou de filiation.

Les solutions de l'art antérieur ne permettent pas de comparer les profils génétiques d'une population connue d'individus au profil génétique de l'échantillon biologique prélevé dans l'environnement pour en retrouver l'individu à l'origine, ni son propriétaire.

### Solution apportée par l'invention

Afin de remédier aux inconvénients de l'art antérieur, l'invention propose dans son acceptation la plus générale un procédé d'identification d'un chien par l'analyse d'un échantillon biologique canin consistant en une déjection canine inconnue abandonnée sur l'espace public, et comparaison avec le profil génétique d'une population connue de chiens enregistrée dans une base de données. Le procédé de l'invention est défini par la revendication 1 et comporte des étapes de :
- prélèvement de l'échantillon biologique
- extraction de l'ADN depuis ledit échantillon
- amplification d'au moins une séquence de l'ADN extrait
- dénaturation et détermination de la taille de l'amplicon
- et comparaison avec les séquences enregistrées dans ladite base de données des profils génétiques de la population canine connue,
caractérisé en ce que l'étape d'amplification est réalisée à l'aide d'un mix de couples d'amorces comprenant l'ensemble des couples pour détecter les loci FH2054, FH2010, PEZ16, FH3313, PEZ6, PEZ8, PEZ12, FH2079, FH2088, PEZ17 et FH2289, à savoir :
- les amorces SEQ ID NO 3 et SEQ ID NO 4 pour le locus FH2054;
- les amorces SEQ ID NO 5 et SEQ ID NO 6 pour le locus FH2010;
- les amorces SEQ ID NO 7 et SEQ ID NO 8 pour le locus PEZ16;
- les amorces SEQ ID NO 9 et SEQ ID NO 10 pour le locus FH3313;
- les amorces SEQ ID NO 11 et SEQ ID NO 12 pour le locus PEZ6;
- les amorces SEQ ID NO 13 et SEQ ID NO 14 pour le locus PEZ8;
- les amorces SEQ ID NO 15 et SEQ ID NO 16 pour le locus PEZ12;
- les amorces SEQ ID NO 17 et SEQ ID NO 18 pour le locus FH2079;
- les amorces SEQ ID NO 19 et SEQ ID NO 20 pour le locus FH2088;
- les amorces SEQ ID NO 21 et SEQ ID NO 22 pour le locus PEZ17; et
- les amorces SEQ ID NO 23 et SEQ ID NO 24 pour le locus FH2289,
et en ce que la température d'hybridation réalisée pendant l'amplification est de 60°C.

Avantageusement, la base de données de profils génétiques de chiens connus à partir d'un échantillon biologique est générée au préalable, lesdits profils génétiques étant établis par le procédé défini par la revendication 2 comprenant:
- prélèvement d'un échantillon biologique sur une population de chiens connus
- extraction de l'ADN depuis ledit échantillon
- amplification d'au moins une séquence de l'ADN extrait
- dénaturation et détermination de la taille de l'amplicon
- listage des profils génétiques des chiens connus dans la base de données
- attribution d'un identifiant dans la base de données au profil génétique d'un chien connu, ledit identifiant comprenant les coordonnées du propriétaire dudit chien connu,
caractérisé en ce que l'étape d'amplification est réalisée à l'aide d'un mix de couples d'amorces comprenant l'ensemble des couples pour détecter les loci FH2054, FH2010, PEZ16, FH3313, PEZ6, PEZ8, PEZ12, FH2079, FH2088, PEZ17 et FH2289, ces couples d'amorces étant tels que définis à la revendication 1.

La base de données de profils génétiques d'une population connue de chiens peut être une base de données préétablie. Ladite base de données est établie par analyse génétique conformément à l'invention d'un échantillon biologique prélevé directement sur un animal comme un prélèvement buccal. Les profils génétiques contenus dans ladite base de données comprennent les séquences nucléotidiques et la taille des amplicons obtenus avec les couples d'amorces selon l'invention. Ces analyses sont référencées dans une base de données qui peut notamment être associée directement ou indirectement au propriétaire de l'animal en question par un identifiant, anonyme ou non.

On entend par amplicon une séquence amplifiée d'ADN par une technique d'amplification ciblée de l'ADN comme la réaction en chaine par polymérase (PCR).

L'échantillon biologique canin s'entend comme une déjection abandonnée sur l'espace public (voirie, trottoir...).

L'étape d'amplification de l'ADN met en oeuvre un réactif comportant un mix de N couples d'amorces tel que défini par la revendication 1.

Les amorces sont de courtes séquences nucléotidiques, généralement d'une vingtaine de nucléotides dont la séquence est complémentaire au brin matrice à amplifier. L'extrémité 3' des amorces permet à une enzyme, la polymérase, de se fixer pour initier l'amplification d'un nouveau brin d'ADN qui sera complémentaire du brin matrice. On entend par couple d'amorces une paire constituée d'une amorce sens et d'une amorce antisens dont l'appariement au brin matrice délimite la séquence d'ADN à amplifier. Le produit d'amplification est appelé amplicon et comprend n copies complémentaires du brin matrice.

Selon un mode préféré de réalisation, au moins une des séquences amplifiées est caractéristique du sexe et les séquences amplifiées comportent au moins une répétition d'un motif d'au moins quatre nucléotides.

Les séquences à amplifier cibles, à l'exception de celle pour l'identification du sexe, sont comprises dans des régions non codantes de l'ADN plus connues sous le nom de microsatellite. Ces régions sont très stables au sein d'une espèce mais présentent une hypervariabilité d'un individu à l'autre. Ces séquences microsatellites ou STR (Short Tandem Repeat) sont composées de répétitions d'un motif de quelques nucléotides en tandem flanquées de deux séquences spécifiques du locus du microsatellite. Le nombre de ces répétitions est caractéristique d'un individu donné et constitue un marqueur génétique communément appelé allèle.

La séquence à amplifier pour l'identification du sexe du chien peut être localisée dans tout gène permettant de déterminer le sexe mâle ou femelle de l'individu.

Avantageusement, l'étape d'amplification de l'ADN met en oeuvre au moins un couple d'amorces pour l'identification du sexe, notamment par l'amplification d'un fragment du gène SRY par un couple d'amorces ayant pour séquence sens SEQ ID NO 1 :
GAA CGC ATT CTT GGT GTG GTC TC
et pour séquence antisens SEQ ID NO 2 :
GGC CAT TTT TCG GCT TCT GTA AG.

L'étape d'amplification de l'ADN selon l'invention met en oeuvre notamment un couple d'amorces spécifiques de la région FH2054, ladite amorce sens ayant pour séquence SEQ ID NO 3 :
GCC TTA TTC ATT GCA GTT AGG G,
ladite amorce antisens ayant pour séquence SEQ ID NO 4 :
ATG CTG AGT TTT GAA CTT TCC C,
ladite région FH2054 comprenant au moins une répétition du motif GATA.

L'on comprend que l'amplicon obtenu à partir de ce couple d'amorce peut avoir une séquence comportant un motif GATA ou une succession de x répétition en tandem de ce motif GATA, où x est un chiffre entier supérieur ou égal à 1. Le nombre de ces répétitions définit un allèle spécifique d'un individu.

L'on comprend que pour chacun des loci suivants, l'amplicon comprend x répétitions du motif correspondant, où x est un chiffre entier supérieur ou égal à 1.

L'étape d'amplification de l'ADN selon l'invention met en oeuvre notamment un couple d'amorces spécifiques de la région FH2010, ladite amorce sens ayant pour séquence SEQ ID NO 5 :
AAA TGG AAC AGT TGA GCA TGC,
ladite amorce antisens ayant pour séquence SEQ ID NO 6 :
CCC CTT ACA GCT TCA TTT TCC,
ladite région FH2010 comprenant au moins une répétition du motif ATGA.

L'étape d'amplification de l'ADN selon l'invention met en oeuvre notamment un couple d'amorces spécifiques de la région PEZ16, ladite amorce sens ayant pour séquence SEQ ID NO 7 :
GCT CTT TGT AAA ATG ACC TG,
ladite amorce antisens ayant pour séquence SEQ ID NO 8 :
GTG GGA ATC GTC CTA AAA CCC,
ladite région PEZ16 comprenant au moins une répétition du motif AAAG.

L'étape d'amplification de l'ADN selon l'invention met en oeuvre notamment un couple d'amorces spécifiques de la région FH3313, ladite amorce sens ayant pour séquence SEQ ID NO 9 :
TGC ACA CCC AAA AAG TAA GC,
ladite amorce antisens ayant pour séquence SEQ ID NO 10 :
CAA TCT GAA GCC AAT CTC ATC,
ladite région FH3313 comprenant au moins une répétition du motif GAAA.

L'étape d'amplification de l'ADN selon l'invention met en oeuvre notamment un couple d'amorces spécifiques de la région PEZ6, ladite amorce sens ayant pour séquence SEQ ID NO 11 :
ATG AGC ACT GGG TGT TAT AC,
ladite amorce antisens ayant pour séquence SEQ ID NO 12 :
ACA CAA TTG CAT TGT CAA AC,
ladite région PEZ6 comprenant au moins une répétition du motif AAAT.

L'étape d'amplification de l'ADN selon l'invention met en oeuvre notamment un couple d'amorces spécifiques de la région PEZ8, ladite amorce sens ayant pour séquence SEQ ID NO 13 :
TAT CGA CTT TAT CAC TGT GG,
ladite amorce antisens ayant pour séquence SEQ ID NO 14 :
ATG GAG CCT CAT GTC TCA TC,
ladite région PEZ8 comprenant au moins une répétition du motif AAAT. L'étape d'amplification de l'ADN selon l'invention met en oeuvre notamment
un couple d'amorces spécifiques de la région PEZ12, ladite amorce sens ayant pour séquence SEQ ID NO 15 :
GTA GAT TAG ATC TCA GGC AG,
ladite amorce antisens ayant pour séquence SEQ ID NO 16 :
TAG GTC CTG GTA GGG TGT GG,
ladite région PEZ12 comprenant au moins une répétition du motif AAAG.

L'étape d'amplification de l'ADN selon l'invention met en oeuvre notamment un couple d'amorces spécifiques de la région FH2079, ladite amorce sens ayant pour séquence SEQ ID NO 17 :
CAG CCG AGC ACA TGG TTT,
ladite amorce antisens ayant pour séquence SEQ ID NO 18 :
ATT GAT TCT GAT ATG CCC AGC,
ladite région FH2079 comprenant au moins une répétition du motif GGAT.

L'étape d'amplification de l'ADN selon l'invention met en oeuvre notamment
un couple d'amorces spécifiques de la région FH2088, ladite amorce sens ayant pour séquence SEQ ID NO 19 :
CCC TCT GCC TAC ATC TCT GC,
ladite amorce antisens ayant pour séquence SEQ ID NO 20 :
TAG GGC ATG CAT ATA ACC AGC,
ladite région FH2088 comprenant au moins une répétition du motif TTTA(m)TTCA(n) où m et n sont des nombres entiers supérieurs ou égaux à un.

L'on comprend que l'amplicon obtenu à partir de ce couple d'amorce peut contenir au moins un motif TTTA suivi d'au moins un motif TTCA. Lorsque m et n sont égaux à 1, l'amplicon comprend une répétition unique d'un motif TTTATTCA.

L'étape d'amplification de l'ADN selon l'invention met en oeuvre notamment un couple d'amorces spécifiques de la région PEZ17, ladite amorce sens ayant pour séquence SEQ ID NO 21 :
CTA AGG GAC TGA ACT TCT CC,
ladite amorce antisens ayant pour séquence SEQ ID NO 22 :
GTG GAA CCT GCT TAA GAT TC,
ladite région PEZ17 comprenant au moins une répétition du motif AAAG.

L'étape d'amplification de l'ADN selon l'invention met en oeuvre notamment un couple d'amorces spécifiques de la région FH2289, ladite amorce sens ayant pour séquence SEQ ID NO 23 :
CAT GGT CTC AGG ATC CTA GGA,
ladite amorce antisens ayant pour séquence SEQ ID NO 24 :
CTA AGC ATT CTC TCT GAT GGT CTT,
ladite région FH2289 comprenant au moins une répétition du motif CTTT.

Selon une variante de réalisation, l'étape d'amplification de l'ADN met en oeuvre en outre des couples d'amorces pour la détection des marqueurs validés par l'ISAG (International Society for Animal Genetics) pour la détection des locus ISAG suivants : AHT121, INU030, REN54P11, AHTk253, AHTk211, REN64E19, FH2848, AHTH130, REN169O18, Amelogenin, INRA21, REN169D01, INU005, INU055, REN105L03, AHT137, AHTh171, REN247M23, CXX279, FH2054, REN162C04 et AHTh260. Dans cette variante de réalisation, les amorces pouvant être utilisées ainsi que la longueur des amplicons sont résumées dans la table 1. Bien entendu, tous couples d'amorces déterminés par l'homme du métier pour détecter ces microsatellites peuvent être utilisés. Cette liste n'est pas limitative et évolue avec les critères ISAG.

**Table 1. Liste des microsatellites ISAG et des amorces d'amplification possibles ainsi que la taille de l'amplicon**

| Locus | Primer | | pb |
|---|---|---|---|
| | | | |
| AHT121 | sens | 5' TAT TgC gAA TgT CAC TgC TT 3' | 68-118 |
| | anti-sens | 5' ATA gAT ACA CTC TCT CTC Cg 3' | |
| INU030 | sens | 5' ggC TCC ATg CTC AAg TCT gT 3' | 139-157 |
| | anti-sens | 5' CAT TgA AAg ggA ATg CTg gT 3' | |
| REN54P11 | sens | 5' ggg ggA ATT AAC AAA gCC TgA g 3' | 222-244 |
| | anti-sens | 5' TgC AAA TTC TgA gCC CCA CTg 3' | |
| AHTk253 | sens | 5' ACA TTT gTg ggC ATT ggg gCT g 3' | 277-297 |
| | anti-sens | 5' TgC ACA Tgg Agg ACA AgC ACg C 3' | |
| AHTk211 | sens | 5' TTA gCA gCC gAg AAA TAC gC 3' | 79-101 |
| | anti-sens | 5' ATT CgC CCg ACT TTg gCA 3' | |
| REN64E19 | sens | 5' TgT ATT TTA ATg Tgg CAg TTT 3' | 139-155 |
| | anti-sens | 5' gAC AAg gAC Agg CAA TAC AgT 3' | |
| FH2848 | sens | 5' CAA AAC CAA CCC ATT CAC TC 3' | 222-244 |
| | anti-sens | 5' gTC ACA Agg ACT TTT CTC CT g 3' | |
| AHTH130 | sens | 5' gTT TCT CTC CCT TCg ggT TC 3' | 111-141 |
| | anti-sens | 5' gAC gTg TgT TCA CgC CAg 3' | |
| REN169018 | sens | 5' CAC CCA ACC TgT CTg TTC CT 3' | 150-170 |
| | anti-sens | 5' ACT gTg TgA gCC AAT CCC TT 3 | |
| Amelogenin | sens | 5' gTg CCA gCT CAg CAg CCC gTg gT 3' | 174-218 |
| | anti-sens | 5' TCg gAg gCA gAg gTg gCT gTg gC 3' | |
| 1NRA21 | sens | 5' ATg TAg TTg AgA TTT CTC CTA Cgg 3' | 87-111 |
| | anti-sens | 5' TAA Tgg CTg ATT TAT TTg gTg 3' | |
| REN169D01 | sens | 5' AgT ggg TTg CAA gTg gAA C 3' | 199-221 |
| | anti-sens | 5' AAT AgC ACA TCT TCC CCA C g 3' | |
| INU005 | sens | 5' CTT TCT ACC AgC AAg gTT AC 3' | 102-136 |
| | anti-sens | 5' TTC CCA TTT AAT TgC CTC T 3' | |
| INU055 | sens | 5' CCA ggC gTC CCT ATC CAT CT 3' | 190-216 |
| | anti-sens | 5' gCA CCA CTT Tgg gCT CCT TC 3' | |
| REN105L03 | sens | 5' ggA ATC AAA AgC Tgg CTC TCT 3' | 231-249 |
| | anti-sens | 5' gAg ATT gCT gCC CTT TTT ACC 3' | |
| AHT137 | sens | 5' TAC AgA gCT CTT AAC Tgg gTC C 3' | 126-156 |
| | anti-sens | 5' CCT TgC AAA gTg TCA TTg CT 3' | |
| AHTh171 | sens | 5' Agg TgC AgA gCA CTC ACT CA 3' | 215-239 |
| | anti-sens | 5' CCC ATC CAC AgT TCA gCT TT 3' | |
| REN247M23 | sens | 5' Tgg TAA CAC CAA ggC TTT CC 3' | 258-282 |
| | anti-sens | 5' TgT CTT TTC CAT ggT ggT gA 3' | |
| CXX279 | sens | 5' TgC TCA ATg AAA TAA gCC Agg 3' | 109-133 |
| | anti-sens | 5' ggC gAC CTT CAT TCT CTg AC 3' | |
| FH2054 | sens | 5' gCC TTA TTC ATT gCA gTT Agg g 3' | 150-170 |
| | anti-sens | 5' ATg CTg AgT TTT gAA CTT TCC C 3' | |
| REN162C04 | sens | 5' TTC CCT TTg CTT TAg TA9 gTT TTg 3' | 192-212 |
| | anti-sens | 5' Tgg CTg TAT TCT TTg gCA CA 3' | |
| AHTh260 | sens | 5' CgC TAT ACC CAC ACC Agg AC 3' | 236-254 |
| | anti-sens | 5' CCA CAg Agg AAg ggA TgC 3' | |

Le procédé d'identification d'un chien par l'analyse d'un échantillon biologique canin comporte au moins une étape de détermination de la taille des amplicons et au moins une étape de comparaison desdites tailles avec la taille des séquences constituant les profils génétiques d'une population connue de chiens.

L'on comprend que la détermination de la taille des amplicons nécessite une étape de dénaturation de l'ADN des amplicons suivie d'une étape de marquage puis d'une étape d'évaluation de la taille dudit amplicon par une technique appropriée notamment la migration sur gel, sur capillaire ou sur puce.

Selon une variante de réalisation ne faisant pas partie de l'invention, le procédé d'identification d'un chien par l'analyse d'un échantillon biologique canin comporte au moins une étape de dénaturation et de séquençage des amplicons pour l'identification de la succession de nucléotides des amplicons et au moins une étape de comparaison des séquences de nucléotides des amplicons à ladite base de données des profils génétiques de la population canine connue.

La description décrit également le procédé d'identification d'un chien par l'analyse d'un échantillon biologique canin comporte une étape de comparaison des amplicons avec une base de données des chiens dangereux.

On entend par chiens dangereux les chiens référencés comme tel et pouvant faire l'objet de restrictions de détention et comprenant les chiens d'attaque, les chiens de garde et de défense et toutes races s'y apparentant. La présente invention permet l'identification du chien responsable d'un dommage, notamment une morsure dès lors qu'une trace d'ADN est disponible.

La description se rapporte également à une variante dans laquelle un même identifiant anonyme est attribué à l'échantillon biologique et aux données génétiques.

L'on entend par données génétiques la taille des amplicons obtenue conformément à l'invention à partir d'un échantillon biologique.

La description décrit également une mallette de prélèvement d'un échantillon biologique canin consistant en une déjection canine inconnue abandonnée sur l'espace public pour la mise en oeuvre du procédé. La mallette comporte :
- des moyens de protection de l'utilisateur, tels que des gants par exemple ;
- des moyens d'humidification de l'échantillon et/ou des écouvillons, tels qu'un spray d'eau ou de solution aqueuse ;
- des écouvillons de prélèvement de l'échantillon. Lesdits écouvillons consistent en une tige sur laquelle est montée une tête amovible en coton ou en papier whatman en forme de peigne pour brosser la déjection canine et prélever les cellules à la surface de la déjection, dont des cellules épithéliales comportant du matériel génétique. La tête amovible permet à l'utilisateur de ne pas contaminer la tête lors de la manipulation ;
- des contenants pré-remplis d'une solution tampon protectrice de l'ADN. Ces contenants sont par exemple des tubes à fermeture hermétique dans lesquels seront éjectées les têtes amovibles des écouvillons porteuses du prélèvement. De préférence, un contenant reçoit une seule tête d'écouvillon. La solution tampon contient de la protéinase K pour protéger l'ADN de dégradation ultérieure. L'ADN peut ainsi être conservé à température ambiante le temps de l'acheminement de l'échantillon. La solution tampon peut avoir la composition suivante :
   ∘ Tris-HCl 1M à pH=7,5
   ∘ EDTA 0,5M à pH=8
   ∘ NaCl 5M
   ∘ SDS 20%
   ∘ Eau déminéralisée
   ∘ Protéinase K à 20 mg/mL ;
- des moyens d'identification des contenants et des données de collecte de l'échantillon, tels que des étiquettes ou des codes barres ou des flashcodes ainsi que des cases pour inscrire le lieu de prélèvement, l'heure, l'état de la déjection canine, la météo au moment du prélèvement... ;
- des sachets de déchets, de préférence zipés.

La présente invention porte également sur un kit d'identification d'un chien pour la mise en oeuvre du procédé caractérisé en ce qu'il comporte :
- un mix de couples d'amorces comprenant l'ensemble des couples pour détecter les loci FH2054, FH2010, PEZ16, FH3313, PEZ6, PEZ8, PEZ12, FH2079, FH2088, PEZ17 et FH2289, ces couples d'amorces étant tels que définis à la revendication 1,
- un tampon d'amplification comportant les réactifs nécessaires à une amplification d'ADN
- un mix de ladder.

On entend par réactifs tous les produits nécessaires pour la mise en oeuvre d'une amplification d'ADN par réaction en chaine par polymérase (PCR), classiquement et non limitativement, une enzyme polymérase, des dNTPs, des sels, des tampons, de l'eau.

On entend par ladder un mix comprenant une pluralité de produits de taille et de poids moléculaire connus pour la mise en place d'une gamme étalon pour le calibrage et la détermination des tailles des amplicons.

Selon une variante ne faisant pas partie de l'invention, le mix de N couples d'amorces comprend au moins trois couples d'amorces choisi parmi les couples énumérés précédemment.

L'on comprend que les couples d'amorces sont choisis parmi les couples d'amorces identifiés par les SEQ ID NO 1 à SEQ ID NO 24 pour l'amplification d'un fragment du gène SRY, et des loci FH2054, FH2010, PEZ16, FH3313, PEZ6, PEZ8, PEZ12, FH2079, FH2088, PEZ17 et FH2289.

Selon l'invention, le mix de couples d'amorces comprend l'ensemble des couples pour détecter les loci FH2054, FH2010, PEZ16, FH3313, PEZ6, PEZ8, PEZ12, FH2079, FH2088, PEZ17 et FH2289.

Selon une variante de la description, ces couples sont répartis en au moins deux mix de multiplex afin d'optimiser la détection des microsatellites d'intérêt.

Selon une autre variante de la description, le kit comprend au moins deux mix multiplex de couples d'amorces pour la détection des loci FH2054, FH2010, PEZ16, FH3313, PEZ6, PEZ8, PEZ12, FH2079, FH2088, PEZ17, FH2289 et pour la détection des loci ISAG, par exemple ceux énumérés en table 1.

Avantageusement, le ladder comprend une pluralité de séquences d'ADN de taille croissante et connue.

L'on comprend que le ladder comprend une pluralité de séquences nucléotidiques de taille connue et croissante comprise entre 35 et 500 paires de bases, et préférentiellement entre 80 et 450 praires de bases.

La présente invention porte également sur un système comportant au moins une base de données d'une population connue de chiens selon l'une des revendications 1 à 7 et des kits d'identification d'un chien dans un échantillon biologique selon la revendication 8, ledit système d'identification comprenant des moyens d'alignement d'une pluralité de séquences, des moyens de comparaison de la taille des séquences et des moyens d'identification du profil génétique d'un chien connu dans ladite base de données, ledit profil génétique correspondant aux séquences amplifiées dudit échantillon biologique, caractérisé en ce que lesdites séquences amplifiées sont spécifiques des loci FH2054, FH2010, PEZ16, FH3313, PEZ6, PEZ8, PEZ12, FH2079, FH2088, PEZ17 et FH2289, les couples d'amorces correspondant à ces locus étant tels que définis à la revendication 1.

Lorsque la base de données d'une population connue de chien a été établie conformément à l'invention, l'on comprend que la correspondance entre les caractéristiques des amplicons de l'échantillon biologique et les références d'un chien de la base de données sera donc importante et optimale.

Selon une variante de la description, un chien dans ladite base de données est identifié par un numéro anonyme auquel correspondent les identifiants de son propriétaire.

Les identifiants du propriétaire du chien peuvent être directement intégrés dans la base de données d'une population connue de chiens ou dans une base séparée.

Selon une autre variante, le système d'identification d'un chien comportant une base de données d'une population connue de chiens et des kits d'identification d'un chien à partir d' un échantillon biologique comporte un générateur d'alerte.

Le système peut générer des alertes à l'utilisateur pour lui notifier les résultats de comparaison entre l'échantillon biologique et les bases. Le système peut également générer automatiquement des avertissements, amendes ou procès-verbaux à destination du propriétaire du chien incriminé en fonction du nombre d'identifications du chien sur une période donnée.

### Description

La présente invention sera mieux comprise à la lumière de la description d'un exemple non limitatif de réalisation : l'identification du chien à l'origine d'une déjection prélevée dans l'environnement, préférentiellement sur la voie publique telle qu'un trottoir par comparaison à une base de profils génétiques de chiens connus, lesdits profils génétiques étant obtenus conformément à l'invention.

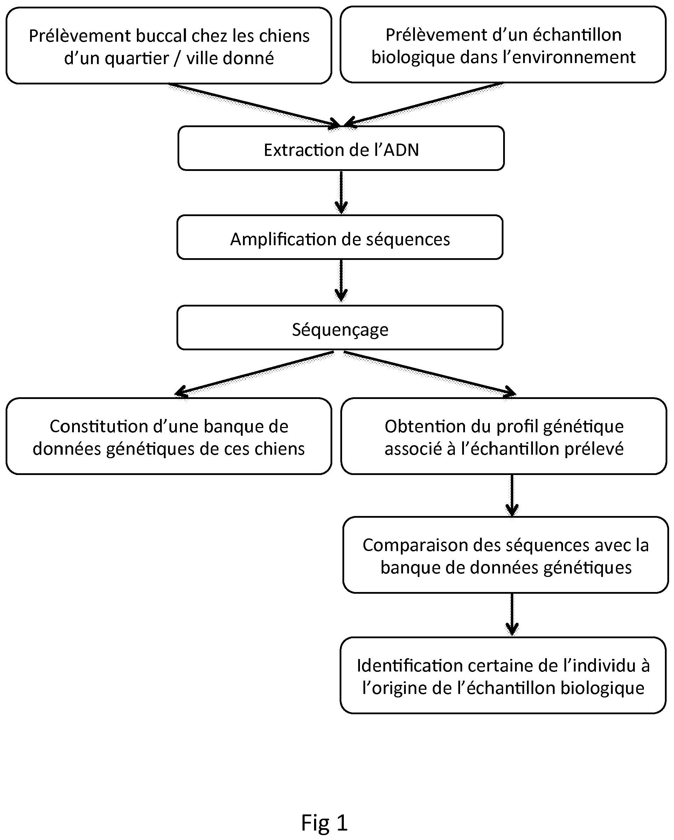

La figure 1 est une représentation schématique du procédé conforme à l'invention.
La figure 2 est un exemple de résultat d'empreinte génétique obtenue à partir d'un prélèvement buccal d'un chien connu.
La figure 3 est un exemple de résultat d'empreinte génétique obtenue à partir d'une déjection canine.

Le listage des séquences présente les séquences décrites dans la présente invention.

### Détermination des loci pour la discrimination d'individus :

La discrimination d'individus canins au sein d'une même espèce et d'une même lignée est réalisée par l'identification de séquences d'ADN microsatellites. Ces microsatellites se composent de répétitions d'un motif de quelques nucléotides en tandem flanquées de deux séquences spécifiques du locus du microsatellite. Le nombre de ces répétitions est caractéristique d'un individu donné et constitue un marqueur génétique, un allèle.

Afin d'augmenter la robustesse du test génétique, onze microsatellites ont été identifiés comme étant suffisamment discriminant pour la détermination de l'appartenance d'un échantillon biologique à un chien donné. Ces microsatellites ont été sélectionnés pour répondre aux contraintes suivantes :
- ils devaient être utilisables en multiplex
- leur taille ne devait pas être trop importante, si possible inférieure à 400 paires de bases pour être sur des petits fragments d'ADN et ainsi être détectables sur de l'ADN potentiellement dégradé
- les STR devaient avoir un fort pouvoir discriminant et également réduire les faux positifs en cas de dégradation de l'ADN de départ, d'où la préférence pour des STR au moins tétranucléotidiques par rapport à des dinucléotidiques.

- la température d'hybridation des amorces des différents STR en multiplex devait être identique
- les STR devaient pouvoir être détectables par des marqueurs (Dye...) compatibles entre eux pour avoir un niveau de discrimination suffisant

Le sexe du chien est également déterminé par la recherche du gène SRY sur le chromosome Y.

La table 2 ci-dessous liste les loci des microsatellites utilisés, leur numéro de chromosome et le motif répété n fois représentatif de l'allèle.

**Table 2. Liste des loci conformément à l'invention**

| Locus | *Chromosome* | Motif |
|---|---|---|
| FH2054 | *12* | GATA |
| FH2010 | *24* | ATGA |
| PEZ16 | *27* | AAAG |
| FH3313 | *19* | GAAA |
| PEZ6 | *27* | AAAT |
| PEZ8 | *17* | AAAT |
| PEZ12 | *3* | AAAG |
| FH2079 | *24* | GGAT |
| FH2088 | *15* | TTTA(m)TTCA(n) |
| PEZ17 | *4* | AAAG |
| FH2289 | *27* | CTTT |

### Design des amorces pour l'amplification des loci préalablement déterminés :

Les microsatellites sont flanqués de séquences hautement conservées au sein d'une même espèce. Il est donc possible de créer des amorces de PCR (réaction en chaîne par polymérase) spécifiques de ces régions chez le chien pour l'amplification des répétitions du motif du microsatellite. La taille des amplicons sera donc fonction du nombre de motifs répétés et sera différente selon les individus. Le nombre de répétitions du motif étant caractéristique d'un allèle.

Les amorces ont été conçues avec le logiciel Primer3Plus dans le but d'avoir une même température d'hybridation pour toutes les amorces à 60°C. Ils ont ensuite été synthétisés à façon (Life Technologie).

La table 3 ci-dessous liste les amorces retenues pour chaque microsatellite ainsi que le nombre de paires de bases des amplicons. SRY correspond à la recherche du sexe du chien.

**Table 3. Liste des amorces pour chaque loci de l'invention**

| Locus | Seq ID NO | Amorce sens | Seq ID NO | Amorce antisens | bp |
|---|---|---|---|---|---|
| SRY | 1 | GAA CGC ATT CTT GGT GTG GTC TC | 2 | GGC CAT TTT TCG GCT TCT GTA AG | 218 |
| FH2054 | 3 | GCC TTA TTC ATT GCA GTT AGG G | 4 | ATG CTG AGT TTT GAA CTT TCC C | 139-177 |
| FH2010 | 5 | AAA TGG AAC AGT TGA GCA TGC | 6 | CCC CTT ACA GCT TCA TTT TCC | 222-244 |
| PEZ16 | 7 | GCT CTT TGT AAA ATG ACC TG | 8 | GTG GGA ATC GTC CTA AAA CCC | 263-334 |
| FH3313 | 9 | TGC ACA CCC AAA AAG TAA GC | 10 | CAA TCT GAA GCC AAT CTC ATC | 340-446 |
| PEZ6 | 11 | ATG AGC ACT GGG TGT TAT AC | 12 | ACA CAA TTG CAT TGT CAA AC | 166-215 |
| PEZ8 | 13 | TAT CGA CTT TAT CAC TGT GG | 14 | ATG GAG CCT CAT GTC TCA TC | 230-260 |
| PEZ12 | 15 | GTA GAT TAG ATC TCA GGC AG | 16 | TAG GTC CTG GTA GGG TGT GG | 250-317 |
| FH2079 | 17 | CAG CCG AGC ACA TGG TTT | 18 | ATT GAT TCT GAT ATG CCC AGC | 263-299 |
| FH2088 | 19 | CCC TCT GCC TAC ATC TCT GC | 20 | TAG GGC ATG CAT ATA ACC AGC | 94-150 |
| PEZ17 | 21 | CTA AGG GAC TGA ACT TCT CC | 22 | GTG GAA CCT GCT TAA GAT TC | 191-245 |
| FH2289 | 23 | CAT GGT CTC AGG ATC CTA GGA | 24 | CTA AGC ATT CTC TCT GAT GGT CTT | 260-350 |

### Extraction de l'ADN des échantillons biologiques (prélèvement buccal, excrément...) :

### - du prélèvement buccal :

Dans cet exemple non limitatif de réalisation, il est préalablement constitué une banque de profils génétiques des chiens d'un quartier ou d'une ville. Pour se faire, les chiens sont soumis à un prélèvement buccal classique afin d'obtenir des cellules de la paroi buccale pour en extraire l'ADN. L'ADN est ensuite extrait par des techniques classiques, préférentiellement par billes magnétiques selon les instructions du fabricant, par exemple, le kit EZ1 DNA Investigator (Qiagen). En synthèse, les cellules sont lysées sous agitation à 800 rpm à 56°C pendant 15 minutes par un tampon de lyse additionné de protéinase K. Le surnageant après centrifugation à 13000 rpm pendant 2 minutes est transféré sur un filtre (DNA IQ Spin-Basket for DNA isolation system - Promega) puis le tout est centrifugé à 13000 rpm 5 minutes et purifié (kit EZ1 DNA Investigator (Qiagen)).

### - de la déjection canine :

Les déjections canines, comme tout excrément, contiennent de l'ADN, *a minima* sous forme de traces. Pour la mise en oeuvre de l'invention, les déjections doivent être de moins de 8 jours et préférentiellement de moins de 48h. Passé ces délais, l'ADN est trop dégradé par les attaques de l'environnement, ce qui rend l'analyse plus délicate et les résultats difficilement interprétables. La déjection canine trouvée sur la voie publique est prélevée à l'aide de la mallette préalablement décrite par l'utilisateur. L'utilisateur humidifie la tête d'un écouvillon et gratte la surface de la déjection avec la tête. Puis l'utilisateur expulse la tête de l'écouvillon dans le tube contenant la solution tampon et identifie le tube. L'utilisateur renseigne également la fiche de renseignement de la mallette en indiquant le lieu de prélèvement de la déjection canine, les conditions météorologiques, l'état de la déjection, l'heure... L'ADN est ensuite extrait par des techniques classiques, préférentiellement par billes magnétiques selon les instructions du fabricant, par exemple, le kit EZ1 DNA Investigator (Qiagen) comme précédemment.

### Amplification par PCR des loci :

Les amorces utilisées pour l'amplification des loci d'intérêt sont reçues lyophilisées du fournisseur (Life technologie) et sont réhydratées à une concentration stock de 10 µM puis conservées aliquotées à -20°C.

Le mix d'amplification est composé de 1,5 µL d'ADN de chien préalablement préparé, de 6 µL d'un mix d'amorces pour l'amplification des séquences désirées et 7,5 µL d'un mix PCR comprenant l'enzyme. Le mix PCR est un mix classique, préférentiellement le Type-it Multiplex PCR Master Mix 2X (Qiagen).

La réaction de PCR est réalisée selon un protocole classique en 30 cycles dans un thermocycler.

La table 4 ci-dessous résume le protocole d'amplification par PCR :

**Table 4. Protocole d'amplification par PCR**

| Dénaturation initiale | 30 cycles | | | Elongation finale |
|---|---|---|---|---|
| | Dénaturation | Hybridation | Elongation | |
| 95°C | 95°C | 60°C | 72°C | 60°C |
| 15 min | 30 sec | 90 sec | 30 sec | 30 min |

Le mix d'amorces comporte les éléments présentés dans la table 5 ci-après:

**Table 5. Composition du multiplex d'amorces**

| Nom du locus | Volume en µL de solution mère à 10 µM |
|---|---|
| FH2054 | 3 |
| FH2010 | 5 |
| PEZ16 | 10 |
| FH3313 | 30 |
| PEZ6 | 4 |
| PEZ8 | 15 |
| PEZ12 | 5 |
| SRY | 5 |
| FH2079 | 9 |
| FH2088 | 5 |
| PEZ17 | 5 |
| FH2289 | 15 |
| Volume final | 111 |

### Dénaturation et révélation des amplicons :

Les amplicons sont ensuite dénaturés. 1,5 µL de produit de PCR sont ajoutés à 24 µL de Hl-Di formamide (Life Technologies) et 1 µL d'un standard de taille (GeneScan-500 LIZ Size Standart - Life Technologies). Ce mix est placé 3 minutes à 95°C puis immédiatement dans la glace.

La révélation est ensuite réalisée en plaçant les mix dans un appareil de lecture de fluorescence tel qu'un séquenceur de type ABI3500 (Life Technologies) fonctionnant avec le logiciel de lecture Genemapper (Life Technologies).

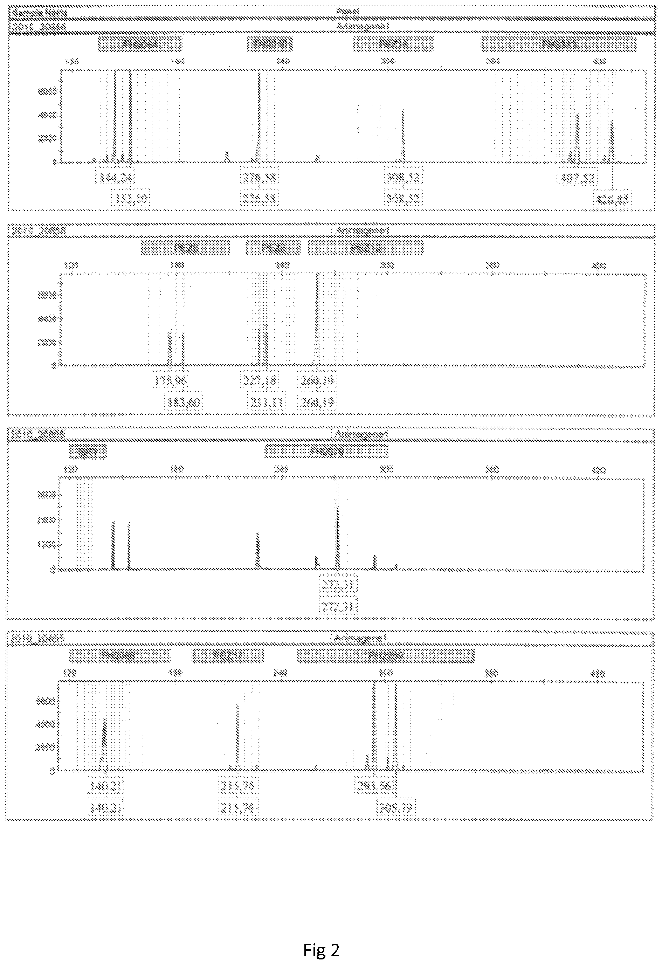

Un exemple de profils obtenus à partir d'un échantillon correspondant à un prélèvement buccal d'un chien connu est présenté en figure 2.

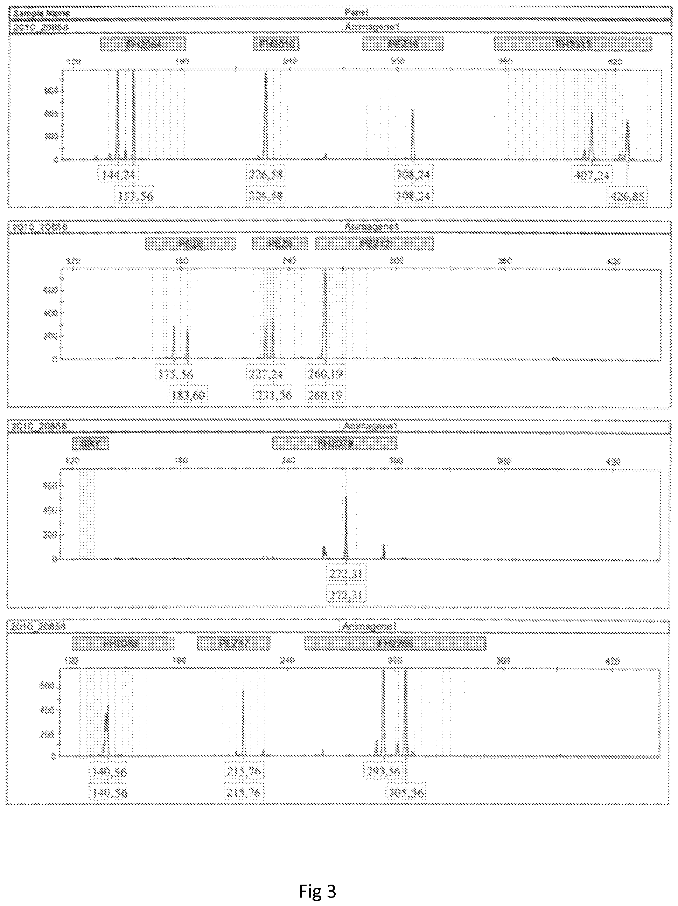

Un exemple de profils obtenus à partir d'une déjection canine d'origine inconnue est présenté en figure 3.

### Génération d'une base de données de profils génétiques de chiens connus à partir des prélèvements buccaux :

Les profils génétiques des chiens ayant subi des prélèvements buccaux sont ensuite répertoriés dans une base de données. Cette base identifie le chien par un numéro auquel est associé son profil génétique et les coordonnées de son propriétaire.

### Alignement et comparaison de séquences provenant de l'échantillon biologique prélevé dans l'environnement à la base de données de profils génétiques de chiens connus pour identification du chien à l'origine de l'excrément :

Le profil génétique de l'échantillon biologique (la déjection canine prélevée sur la voie publique) est comparé aux profils génétiques référencés dans la base de données ci-avant mentionnée.

Si aucune correspondance solide n'est trouvée, alors la déjection canine n'a pas pour origine un chien référencé dans la base. Son origine est donc inconnue.

Si une correspondance est établie, cela signifie que la déjection canine appartient à un chien connu. Par conséquent, le propriétaire du chien incriminé peut être retrouvé afin de lui appliquer une sanction tel un avertissement, un procès-verbal ou une amende.

L'on remarque que les profils en figure 3 obtenus à partir d'une déjection canine inconnue correspondent en tout point aux profils en figure 2 du prélèvement buccal du chien connu. Il s'agit donc du même individu canin.

### Références bibliographiques :

Halverson J. et Basten C., A PCR multoplex and database for forensic DNA identification of dogs, J Forensic Sci., 2005 Mar; 50(2):352-63
Tom B. K. et al, Development of a nomenclature system for a canine STR multiplex reagent kit, J Forensic Sci. 2010 May; 55(3):597-604
Wictum E. et al, Development validation of dogfiler, a novel multiplex for canine DNA profiling in forensic casework, Forensic Sci Int Genet., 2013 Jan; 7(1):82-91

## Revendications

1. Procédé d'identification d'un chien par l'analyse d'un échantillon biologique canin consistant en une déjection canine inconnue abandonnée sur l'espace public, et comparaison avec le profil génétique d'une population connue de chiens enregistrée dans une base de données, comprenant les étapes de :
- prélèvement de l'échantillon biologique
- extraction de l'ADN depuis ledit échantillon
- amplification d'au moins une séquence de l'ADN extrait
- dénaturation et détermination de la taille de l'amplicon
- et comparaison avec les séquences enregistrées dans ladite base de données des profils génétiques de la population canine connue,
**caractérisé en ce que** l'étape d'amplification est réalisée à l'aide d'un mix de couples d'amorces comprenant l'ensemble des couples pour détecter les loci FH2054, FH2010, PEZ16, FH3313, PEZ6, PEZ8, PEZ12, FH2079, FH2088, PEZ17 et FH2289, à savoir :
- les amorces SEQ ID NO 3 et SEQ ID NO 4 pour le locus FH2054;
- les amorces SEQ ID NO 5 et SEQ ID NO 6 pour le locus FH2010;
- les amorces SEQ ID NO 7 et SEQ ID NO 8 pour le locus PEZ16;
- les amorces SEQ ID NO 9 et SEQ ID NO 10 pour le locus FH3313;
- les amorces SEQ ID NO 11 et SEQ ID NO 12 pour le locus PEZ6;
- les amorces SEQ ID NO 13 et SEQ ID NO 14 pour le locus PEZ8;
- les amorces SEQ ID NO 15 et SEQ ID NO 16 pour le locus PEZ12;
- les amorces SEQ ID NO 17 et SEQ ID NO 18 pour le locus FH2079;
- les amorces SEQ ID NO 19 et SEQ ID NO 20 pour le locus FH2088;
- les amorces SEQ ID NO 21 et SEQ ID NO 22 pour le locus PEZ17; et
- les amorces SEQ ID NO 23 et SEQ ID NO 24 pour le locus FH2289,
et **en ce que** la température d'hybridation réalisée pendant l'amplification est de 60°C.

2. Procédé d'identification d'un chien par l'analyse d'un échantillon biologique canin consistant en une déjection canine inconnue abandonnée sur l'espace public, et comparaison avec le profil génétique d'une population connue de chiens enregistrée dans une base de données selon la revendication 1 comprenant la génération au préalable de la base de données de profils génétiques de chiens connus à partir d'un échantillon biologique, lesdits profils génétiques étant établis par :
- prélèvement d'un échantillon biologique par prélèvement buccal sur une population de chiens connus
- extraction de l'ADN depuis ledit échantillon
- amplification d'au moins une séquence de l'ADN extrait
- dénaturation et détermination de la taille de l'amplicon
- listage des profils génétiques des chiens connus dans la base de données
- attribution d'un identifiant dans la base de données au profil génétique d'un chien connu, ledit identifiant comprenant les coordonnées du propriétaire dudit chien connu, **caractérisé en ce que** l'étape d'amplification est réalisée à l'aide d'un mix de couples d'amorces comprenant l'ensemble des couples pour détecter les loci FH2054, FH2010, PEZ16, FH3313, PEZ6, PEZ8, PEZ12, FH2079, FH2088, PEZ17 et FH2289, ces couples d'amorces étant tels que définis à la revendication 1.

3. Procédé d'identification d'un chien par l'analyse d'un échantillon biologique canin consistant en une déjection canine inconnue abandonnée sur l'espace public et comparaison avec le profil génétique d'une population connue de chiens enregistrée dans une base de données selon l'une quelconque des revendications 1 à 2 caractérisé en ce ladite étape d'amplification met en outre en oeuvre un couple d'amorce caractéristique du sexe.

4. Procédé d'identification d'un chien par l'analyse d'un échantillon biologique canin consistant en une déjection canine inconnue abandonnée sur l'espace public et comparaison avec le profil génétique d'une population connue de chiens enregistrée dans une base de données selon la revendication 3 **caractérisé en ce que** ledit couple d'amorces caractéristique du sexe permet l'amplification d'un fragment du gène SRY et a pour séquence sens SEQ ID NO 1 :
GAA CGC ATT CTT GGT GTG GTC TC
et pour séquence antisens SEQ ID NO 2:
GGC CAT TTT TCG GCT TCT GTA AG.

5. Procédé d'identification d'un chien par l'analyse d'un échantillon biologique canin selon l'une des revendications précédentes, **caractérisé en ce qu'**il comprend en outre une étape d'amplification à l'aide d'au moins un couple d'amorces pour la détection des marqueurs validés par l'ISAG (International Society for Animal Genetics) pour la détection des locus ISAG choisi parmi AHT121, INU030, REN54P11, AHTk253, AHTk211, REN64E19, FH2848, AHTH130, REN169O18, Amelogenin, INRA21, REN169D01, INU005, INU055, REN105L03, AHT137, AHTh171, REN247M23, CXX279, , REN162C04 et AHTh260, les amorces sens et antisens correspondant auxdits locus étant décrites dans le Tableau 1.

6. Procédé d'identification d'un chien par l'analyse d'un échantillon biologique canin consistant en une déjection canine inconnue abandonnée sur l'espace public et comparaison avec le profil génétique d'une population connue de chiens enregistrée dans une base de données selon l'une quelconque des revendications précédentes **caractérisé en ce qu'**il comporte une étape de comparaison des amplicons avec une base de données des chiens dangereux.

7. Procédé d'identification d'un chien par l'analyse d'un échantillon biologique canin consistant en une déjection canine inconnue abandonnée sur l'espace public et comparaison avec le profil génétique d'une population connue de chiens enregistrée dans une base de données selon l'une quelconque des revendications précédentes **caractérisé en ce qu'**un même identifiant anonyme est attribué à l'échantillon biologique et aux données génétiques.

8. Kit d'identification d'un chien pour la mise en oeuvre du procédé selon l'une quelconque des revendications précédentes **caractérisé en ce qu'**il comprend :
- un mix de couples d'amorces comprenant l'ensemble des couples pour détecter les loci FH2054, FH2010, PEZ16, FH3313, PEZ6, PEZ8, PEZ12, FH2079, FH2088, PEZ17 et FH2289, ces couples d'amorces étant tels que définis à la revendication 1,
- un tampon d'amplification comportant les réactifs nécessaires à une amplification d'ADN,
- un mix de ladder.

9. Système d'identification d'un chien comportant au moins une base de données d'une population connue de chiens selon l'une des revendications 1 à 7 et des kits d'identification d'un chien à partir d' un échantillon biologique selon la revendication 8, ledit système d'identification comprenant des moyens d'alignement d'une pluralité de séquences, des moyens de comparaison de la taille des séquences et des moyens d'identification du profil génétique d'un chien connu dans ladite base de données, ledit profil génétique correspondant aux séquences amplifiées dudit échantillon biologique, **caractérisé en ce que** lesdites séquences amplifiées sont spécifiques des loci FH2054, FH2010, PEZ16, FH3313, PEZ6, PEZ8, PEZ12, FH2079, FH2088, PEZ17 et FH2289, les couples d'amorces correspondant à ces locus étant tels que définis à la revendication 1.

10. Système d'identification d'un chien comportant au moins une base de données d'une population connue de chiens et des kits d'identification d'un chien à partir d' un échantillon biologique selon la revendication 9 **caractérisé en ce qu'**un chien dans ladite base de données est identifié par un numéro anonyme auquel correspondent les identifiants de son propriétaire.

11. Système d'identification d'un chien comportant une base de données d'une population connue de chiens et des kits d'identification d'un chien à partir d' un échantillon biologique selon l'une quelconque des revendications 9 ou 10 **caractérisé en ce qu'**il comporte un générateur d'alerte.

## Patentansprüche

1. Verfahren zur Identifikation eines Hundes durch die Analyse einer biologischen Probe eines Hunds bestehend aus einem im öffentlichen Bereich hinterlassenen unbekannten Hundekot, und den Vergleich mit dem genetischen Profil einer bekannten Hundepopulation, die in einer Datenbank gespeichert ist, welches folgende Schritte umfasst:
- die Entnahme einer biologischen Probe
- die Extraktion der DNA aus besagter Probe
- die Amplifikation von mindestens einer extrahierten DNA-Sequenz
- die Denaturierung und Größenbestimmung des Amplicons
- und der Vergleich mit den Sequenzen, die in besagter Datenbank der genetischen Profile der bekannten Hundepopulation gespeichert sind,
**dadurch gekennzeichnet, dass** der Schritt der Amplifikation mithilfe einer Mischung an Primerpaaren erfolgt, welche alle Paare zur Ermittlung der Loci FH2054, FH2010, PEZ16, FH3313, PEZ6, PEZ8, PEZ12, FH2079, FH2088, PEZ17 und FH2289 umfassen, d.h.:
- die Primer SEQ ID NO 3 und SEQ ID NO 4 für den Locus FH2054;
- die Primer SEQ ID NO 5 und SEQ ID NO 6 für den Locus FH2010;
- die Primer SEQ ID NO 7 und SEQ ID NO 8 für den Locus PEZ16;
- die Primer SEQ ID NO 9 und SEQ ID NO 10 für den Locus FH3313;
- die Primer SEQ ID NO 11 und SEQ ID NO 12 für den Locus PEZ6;
- die Primer SEQ ID NO 13 und SEQ ID NO 14 für den Locus PEZ8;
- die Primer SEQ ID NO 15 und SEQ ID NO 16 für den Locus PEZ12;
- die Primer SEQ ID NO 17 und SEQ ID NO 18 für den Locus FH2079;
- die Primer SEQ ID NO 19 und SEQ ID NO 20 für den Locus FH2088;
- die Primer SEQ ID NO 21 und SEQ ID NO 22 für den Locus PEZ17;
- die Primer SEQ ID NO 23 und SEQ ID NO 24 für den Locus FH2289;
und dadurch, dass die während der Amplifikation hergestellte Hybridisierungs-Temperatur 60 °C beträgt.

2. Verfahren zur Identifikation eines Hundes durch die Analyse einer biologischen Probe bestehend aus einem unbekannten, im öffentlichen Bereich hinterlassenen Hundekot, und Vergleich mit dem genetischen Profil einer bekannten Hundepopulation, die in einer Datenbank gespeichert ist gemäß Anspruch 1, welches vorab die Generierung der Datenbank bekannter genetischer Hundeprofile ausgehend von einer biologischen Probe umfasst, wobei besagte genetische Profile erstellt werden durch:
- die Entnahme einer biologischen Probe mittels eines Mundabstrichs von einer bekannten Hundepopulation
- die Extraktion der DNA aus besagter Probe
- die Amplifikation von mindestens einer extrahierten DNA-Sequenz
- die Denaturierung und Größenbestimmung des Amplicons
- die Auflistung der genetischen Profile bekannter Hunde in der Datenbank
- die Zuteilung einer Kennung zu dem genetischen Profil eines bekannten Hundes in der Datenbank, wobei besagte Kennung die Anschrift des Besitzers des bekannten Hundes umfasst, **dadurch gekennzeichnet, dass** der Schritt der Amplifikation mithilfe einer Mischung von Primerpaaren erfolgt, die alle Paare zur Ermittlung der Loci FH2054, FH2010, PEZ16, FH3313, PEZ6, PEZ8, PEZ12, FH2079, FH2088, PE217 und FH2289 umfassen, wobei diese Primerpaare die in Anspruch 1 definierten Paare sind.

3. Verfahren zur Identifikation eines Hundes durch die Analyse einer biologischen Probe eines Hundes bestehend aus einem unbekannten Hundekot, der im öffentlichen Bereich hinterlassen wurde, und Vergleich mit dem genetischen Profil einer bekannten Hundepopulation, die in einer Datenbank nach einem der Ansprüche 1 bis 2 gespeichert ist, **dadurch gekennzeichnet, dass** in besagtem Schritt der Amplifikation des Weiteren ein Primerpaar angewendet wird, das für das Geschlecht charakteristisch ist.

4. Verfahren zur Identifikation eines Hundes durch die Analyse einer biologischen Probe eines Hundes bestehend aus einem unbekannten Hundekot, der im öffentlichen Bereich hinterlassen wurde, und Vergleich mit dem genetischen Profil einer bekannten Hundepopulation, die in einer Datenbank nach Anspruch 3 gespeichert ist, **dadurch gekennzeichnet, dass** besagtes geschlechtscharakteristisches Primerpaar die Amplifikation eines Fragments des Gens SRY ermöglicht und folgende Sens-Sequenz SEQ ID NO 1 hat:
- GAA CGC ATT CTT GGT CTG GTC TC
bzw. folgende Antisens-Sequenz SEQ ID NO 2 hat:
- GGC CAT TTT TCG GCT TCT GTA AG.

5. Verfahren zur Identifikation eines Hundes durch die Analyse einer biologischen Hundeprobe nach einem der vorhergehenden Ansprüche, **dadurch gekennzeichnet, dass** es des Weiteren einen Schritt der Amplifikation mithilfe von mindestens einem Primerpaar zur Erkennung der von der ISAG (International Society for Animal Genetics) validierten Marker für die Erkennung der ISAG-Loci umfasst, die ausgewählt werden aus AHT121, INU030, REN54P11, AHTk253, AHTk211, REN64E19, FH2848, AHTH130, REN169O18, Amelogenin, INRA21, REN169D01, INU005, INU055, REN105L03, AHT137, AHTh171, REN247M23, CXX279, REN162C04 und AHTh260, wobei Sens- und Antisens- Primer besagter Loci in Tabelle 1 beschrieben werden.

6. Verfahren zur Identifikation eines Hundes durch die Analyse einer biologischen Probe eines Hundes bestehend aus einem unbekannten Hundekot, der im öffentlichen Bereich hinterlassen wurde, und Vergleich mit dem genetischen Profil einer bekannten Hundepopulation, die in einer Datenbank gespeichert wurde gemäß einem der vorhergehenden Ansprüche, **dadurch gekennzeichnet, dass** es einen Schritt des Vergleichs der Amplicons mit einer Datenbank von gefährlichen Hunden umfasst.

7. Verfahren zur Identifikation eines Hundes durch die Analyse einer biologischen Probe eines Hundes bestehend aus einem unbekannten Hundekot, der im öffentlichen Bereich hinterlassen wurde, und Vergleich mit dem genetischen Profil einer bekannten Hundepopulation, die in einer Datenbank nach einem der vorhergehenden Ansprüche gespeichert wurde, **dadurch gekennzeichnet, dass** der biologischen Probe und den genetischen Daten ein und dieselbe anonyme Kennung zugeordnet werden.

8. Set zur Identifikation eines Hundes für die Anwendung des Verfahrens nach einem der vorhergehenden Ansprüche, **dadurch gekennzeichnet, dass** es Folgendes umfasst:
- eine Mischung von Primerpaaren, welche alle Paare zur Erkennung der Loci FH2054, FH2010, PEZ16, FH3313, PEZ6, PEZ8, PEZ12, FH2079, FH2088, PEZ17 und FH2289 umfassen, wobei diese Paare die in Anspruch 1 definierten Paare sind,
- ein Amplifikationspuffer, welcher die Reaktanten umfasst, die für eine Amplifikation der DNA erforderlich sind,
- eine Ladder-Mischung.

9. System zur Identifikation eines Hundes, welches zumindest eine Datenbank einer bekannten Hundepopulation nach den Ansprüchen 1 bis 7 umfasst und Identifikationssets für einen Hund ausgehend von einer biologischen Probe nach Anspruch 8, wobei besagtes Identifikationssystem Mittel zur Ausrichtung einer Vielzahl von Sequenzen, Mittel zum Vergleich der Größe der Sequenzen und Mittel zur Identifikation des genetischen Profils eines bekannten Hundes in besagter Datenbank umfasst, wobei besagtes genetisches Profil den amplifizierten Sequenzen der besagten biologischen Probe entspricht, **dadurch gekennzeichnet, dass** besagte amplifizierte Sequenzen spezifisch sind für die Loci FH2054, FH2010, PEZ16, FH3313, PEZ6, PEZ8 PEZ12, FH2079, FH2088, PEZ17 und FH2289, wobei die Primerpaare, welchen diesen Loci entsprechen, die in Anspruch 1 definierten Paare sind.

10. System zur Identifikation eines Hundes, welches mindestens eine Datenbank einer bekannten Hundepopulation und Identifikationssets für einen Hund ausgehend von einer biologischen Probe nach Anspruch 9 umfasst, **dadurch gekennzeichnet, dass** ein Hund in besagter Datenbank identifiziert wird durch eine anonyme Nummer, dem die Kennungen seines Besitzers entsprechen.

11. System zur Identifikation eines Hundes, welches eine Datenbank einer bekannten Hundepopulation und Identifikationssets für einen Hund ausgehend von einer biologischen Probe nach einem der Ansprüche 9 oder 10 umfasst, **dadurch gekennzeichnet, dass** es einen Warnungserzeuger umfasst.

## Claims

1. Method for identifying a dog by analysing a canine laboratory sample consisting of an anonymous dog stool left lying in the public space and comparison with the genetic profile of a known population of dogs recorded in a database, comprising the stages of:
- collection of the laboratory sample
- extraction of the DNA from said sample
- amplification of at least one sequence of the extracted DNA
- denaturation and determination of the size of the amplicon
- and comparison with the sequences recorded in said database containing the genetic profiles of the known canine population,
**characterised in that** the amplification stage is performed based on a mix of primer pairs comprising all the pairs for detecting the loci FH2054, FH2010, PEZ16, FH3313, PEZ6, PEZ8, PEZ12, FH2079, FH2088, PEZ17 and FH2289, i.e.:
- the primers SEQ ID NO 3 and SEQ ID NO 4 for the locus FH2054;
- the primers SEQ ID NO 5 and SEQ ID NO 6 for the locus FH2010;
- the primers SEQ ID NO 7 and SEQ ID NO 8 for the locus PEZ16;
- the primers SEQ ID NO 9 and SEQ ID NO 10 for the locus FH3313;
- the primers SEQ ID NO 11 and SEQ ID NO 12 for the locus PEZ6;
- the primers SEQ ID NO 13 and SEQ ID NO 14 for the locus PEZ8;
- the primers SEQ ID NO 15 and SEQ ID NO 16 for the locus PEZ12;
- the primers SEQ ID NO 17 and SEQ ID NO 18 for the locus FH2079;
- the primers SEQ ID NO 19 and SEQ ID NO 20 for the locus FH2088;
- the primers SEQ ID NO 21 and SEQ ID NO 22 for the locus PEZ17; and
- the primers SEQ ID NO 23 and SEQ ID NO 24 for the locus FH2289,
and **in that** the hybridisation temperature applied during amplification is 60°C.

2. Method for identifying a dog by analysing a canine laboratory sample consisting of an anonymous dog stool left lying in the public space and comparison with the genetic profile of a known population of dogs recorded in a database according to claim 1, comprising prior generation of the database of genetic profiles of known dogs based on a laboratory sample, wherein said genetic profiles are established by:
- collection of a laboratory sample by means of a buccal swab from a known dog population
- extraction of the DNA from said sample
- amplification of at least one sequence of the extracted DNA
- denaturation and determination of the size of the amplicon
- listing the genetic profiles of the known dogs in the database
- allocation of an identifier to the genetic profile of a known dog in the database, wherein said identifier comprises the contact details of the owner of said known dog, **characterised in that** the amplification stage is performed using a mix of primer pairs for detecting the loci FH2054, FH2010, PEZ16, FH3313, PEZ6, PEZ8, PEZ12, FH2079, FH2088, PEZ17 and FH2289, said primer pairs being as defined in claim 1.

3. Method for identifying a dog by analysing a canine laboratory sample consisting of an anonymous dog stool left lying in the public space and comparison with the genetic profile of a known population of dogs recorded in a database according to any one of claims 1 to 2, **characterised in that** said amplification stage furthermore employs a primer pair characteristic of gender.

4. Method for identifying a dog by analysing a canine laboratory sample consisting of an anonymous dog stool left lying in the public space and comparison with the genetic profile of a known population of dogs recorded in a database according to claim 3, **characterised in that** said primer pair characteristic of gender enables amplification of a fragment of the SRY gene and has as sense sequence SEQ ID NO 1:
GAA CGC ATT CTT GGT GTG GTC TC
and as antisense sequence SEQ ID NO 2:
GGC CAT TTT TCG GCT TCT GTA AG.

5. Method for identifying a dog by analysing a canine laboratory sample according to any of the preceding claims, **characterised in that** it furthermore comprises an amplification stage using at least one primer pair for detecting the markers validated by the ISAG (International Society for Animal Genetics) for detection of the ISAG loci selected from among AHT121, INU030, REN54P11, AHTk253, AHTk211, REN64E19, FH2848, AHTH130, REN169O18, Amelogenin, INRA21, REN169D01, INU005, INU055, REN105L03, AHT137, AHTh171, REN247M23, CXX279, , REN162C04 and AHTh260, the sense and antisense primers corresponding to said loci being described in Table 1.

6. Method for identifying a dog by analysing a canine laboratory sample consisting of an anonymous dog stool left in the public space and comparison with the genetic profile of a known population of dogs recorded in a database according to any one of claims 1 to 2, **characterised in that** said amplification stage furthermore employs a primer pair characteristic of gender.

7. Method for identifying a dog by analysing a canine laboratory sample consisting of an anonymous dog stool left lying in the public space and comparison with the genetic profile of a known population of dogs recorded in a database according to any one of the preceding claims, **characterised in that** the same anonymous identifier is allocated to the laboratory sample and to the genetic data.

8. Kit for identifying a dog for implementing the method according to any one of the preceding claims, **characterised in that** it comprises:
- a mix of primer pairs comprising all the pairs for detecting the loci FH2054, FH2010, PEZ16, FH3313, PEZ6, PEZ8, PEZ12, FH2079, FH2088, PEZ17 and FH2289, said primer pairs being as defined in claim 1,
- an amplification buffer comprising the reagents required for a DNA amplification,
- a ladder mix.

9. System for identifying a dog, comprising at least one database of a known population of dogs according to any of claims 1 to 7 and kits for identifying a dog based on a laboratory sample according to claim 8, wherein said identification system comprises means of aligning a plurality of sequences, means of comparing the size of the sequences and means of identifying the genetic profile of a known dog in said database, said genetic profile corresponding to the amplified sequences of said laboratory sample, **characterised in that** said amplified sequences are specific to the loci FH2054, FH2010, PEZ16, FH3313, PEZ6, PEZ8, PEZ12, FH2079, FH2088, PEZ17 and FH2289, the primer pairs corresponding to these loci being as defined in claim 1.

10. System for identifying a dog, comprising at least one database of a known population of dogs and kits for identifying a dog based on a laboratory sample according to claim 9, **characterised in that** a dog in said database is identified by an anonymous number to which its owner's identifiers correspond.

11. System for identifying a dog comprising a database of a known population of dogs and kits for identifying a dog based on a laboratory sample according to either of claims 9 or 10, **characterised in that** it comprises an alert generator.
